# EUROPÄISCHE PATENTSCHRIFT

(11) **EP 2 272 467 B1**
(45) Veröffentlichungstag und Bekanntmachung des Hinweises auf die Patenterteilung: **06.09.2017**
(21) Anmeldenummer: 10006869.1
(22) Anmeldetag: 02.07.2010
(51) Int. Cl.: A61F 2/38, A61F 2/00, A61F 2/30

(54) **Überhöhtes Implantat zur Rekonstruktion von Meniskusdefekten oder Meniskusteildefekten**
Inflated implant for reconstructing meniscus defects or minor meniscus defects
Implant surélevé pour la reconstruction de défauts du ménisque ou de défauts partiels du ménisque

(30) Priorität: 06.07.2009 DE 102009032218
(43) Veröffentlichungstag der Anmeldung: 12.01.2011
(73) Patentinhaber: Aesculap AG, 78532 Tuttlingen/Donau (DE)
(72) Erfinder: Jansson, Volkmar, 82205 Gilching (DE); Müller, Peter, 86899 Landsberg am Lech (DE)
(74) Vertreter: Patentanwälte Ruff, Wilhelm, Beier, Dauster & Partner mbB

(56) Entgegenhaltungen:
- WO-A2-03/007788
- DE-U1-202008 013 581
- US-A1- 2008 086 210
- US-A1- 2008 097 606

## Beschreibung

Die vorliegende Erfindung betrifft ein Implantat zur Rekonstruktion von Meniskusdefekten oder Meniskusteildefekten, ein entsprechendes chirurgisches Set sowie ein Verfahren zur Rekonstruktion von Meniskusdefekten oder Meniskusteildefekten.

Die Behandlung von Meniskusdefekten stellt eine der großen Herausforderungen in der Orthopädie und Unfallchirurgie dar. Es galt lange Zeit das Paradigma, dass die Zerstörung der meniskalen Strukturen irreversibel, ihre "Restitutio ad integrum" nicht mehr möglich ist.

Inzwischen gibt es bei der Behandlung von Meniskusdefekten eine Reihe guter Ansätze. Nach wie vor sind bislang jedoch nur wenige geeignete Implantate verfügbar. Zum Teil werden auch Meniskustransplantate von Spenderleichen verwendet. Nachteilig ist jedoch, dass es sich hierbei um totes und daher nur bedingt revitalisierbares Material handelt.

Ein zentrales Problem bei den Meniskusrekonstruktionen stellt die stabile Verankerung des Implantats bzw. Transplantats dar. Da die Durchblutung des Meniskus von der sogenannten Basis, d.h. von der der Gelenkkapsel zugewandten Seite des Meniskus, erfolgt, muss jedes Implantat bzw. Transplantat mit seiner Basis an dieser Gelenkkapsel bzw. Randleiste möglichst fest verankert werden. Kommt es an der Gelenkkapsel nicht zu einer stabilen Verankerung, kann beispielsweise die Revitalisierung eines Transplantats nicht erfolgen. Eine stabile Verankerung in der Phase der Einheilung im Sinne eines engen Kontaktes zwischen dem Meniskustransplantat bzw. -implantat und der Gelenkkapsel muss darüber hinaus auch bei Bewegungen des Gelenkes bestehen bleiben.

Üblicherweise werden für die Meniskusrekonstruktion eingesetzte Im-plantate oder Transplantate mit einer ähnlichen Technik an die Gelenkkapsel fixiert, mit der auch Meniskusrupturen behandelt werden. Bei dieser Technik werden die Implantate bzw. Transplantate mit Fäden eingenäht oder mit geeigneten Ankerelementen an der Gelenkkapsel angeheftet. Dabei werden die Ankerelemente durch den anzuheftenden Meniskus, das anzuheftende Implantat bzw. das anzuheftende Transplantat hindurch in die Gelenkkapsel geschossen. Ein besonderes Problem stellt dabei auch die Fixierung der Endstellen eines Meniskusimplantats oder - transplantats dar. Eine der üblichen Fixierungstechniken besteht darin, die Endstellen mit Fäden zu armieren und die Fäden im tibialen Knochen zu verankern.

Trotz all dieser Bemühungen sind die erzielten Ergebnisse bei der Behandlung von Knorpel- und Meniskusdefekten noch nicht befriedigend. Insbesondere sind die vorstehend beschriebenen Behandlungsmethoden mühsam und zeitaufwändig. Oft muss sogar zumindest eine teilweise Eröffnung des Gelenkes im Sinne einer Arthrotomie erfolgen. Hinzu kommt, dass es technisch oftmals sehr schwierig ist, Ankerelemente mit athroskopischen Mitteln in die gewünschte Position einzubringen. In der US 2008/0086210 A1 greifen Verankerungselemente zur Verankerung des dort beschriebenen Kunstmeniskus als Nut in den tibialen Knochen ein, was intraoperativ schwer durchführbar ist. Hinzu kommt, dass die Verankerung nicht an der Gelenkkapsel sondern an den Gelenkknochen stattfindet. Die US 2008/0086210 A1 zeigt die Merkmale des Oberbegriffs von Anspruch 1. Aus der DE 20 2008 013 581 U1 ist ein vorgespanntes Prothesegerät zum Ersetzen eines geschädigten Meniskus eines Kniegelenks bekannt.

Weitere Meniskusimplantate sind aus den Dokumenten US 2008/0086210 A1, WO 03/007788 A2 sowie US 2008/0097606 A1 bekannt.

Aufgabe der vorliegenden Erfindung ist es daher, ein Implantat für die Meniskusrekonstruktion bereitzustellen, welches eine möglichst stabile Verankerung bzw. Fixierung an der Gelenkkapsel (Randleiste) eines Patienten ermöglicht. Außerdem soll das Implantat möglichst einfach, insbesondere mittels arthroskopischen Methoden, implantierbar sein.

Diese Aufgabe wird erfindungsgemäß gelöst durch ein Implantat mit den Merkmalen des unabhängigen Anspruchs 1. Bevorzugte Ausführungsformen des Implantats sind Gegenstand der abhängigen Ansprüche 2 bis 15. Des Weiteren betrifft die vorliegende Erfindung ein chirurgisches Set mit den Merkmalen des Anspruchs 16.

Bei dem erfindungsgemäßen Implantat handelt es sich um ein Implantat zur Rekonstruktion von Meniskusdefekten oder Meniskusteildefekten, umfassend einen überhöhten Formkörper mit einer femoralen Kondylen-Kontaktfläche, einer tibialen Kondylen-Kontaktfläche und einer Gelenkkapsel-Kontaktfläche, insbesondere nach Art eines Kunstmeniskus oder Kunstteilmeniskus mit einer im Wesentlichen einem natürlichen Meniskus bzw. Teilmeniskus gleichenden Außenflächenkontur, wobei auf der Außenfläche bzw. Oberfläche des überhöhten Formkörpers zumindest eine, insbesondere eine, zusätzliche Überhöhung ausgebildet ist. Ggf. können auch zwei, drei, vier oder mehr zusätzliche Überhöhungen ausgebildet sein.

Unter einem überhöhten Formkörper im Sinne der vorliegenden Erfindung soll ein Formkörper verstanden werden, der eine Überhöhung aufweist. Unter einer Überhöhung im Sinne der vorliegenden Erfindung soll eine Querneigung des Formkörpers verstanden werden. Ist der Formkörper beispielsweise kurvenförmig ausgebildet, wie im Folgenden noch eingehender erläutert wird, so ist die Querneigung bzw. Überhöhung vorzugsweise im Kurvenbereich des überhöhten Formkörpers zum Kurveninneren hin ausgebildet.

Durch die vorliegende Erfindung wird ein Implantat zur Rekonstruktion von Meniskusdefekten oder Meniskusteildefekten auf der Basis eines erhöhten Formkörpers bereitgestellt, der als Kunstmeniskus bzw. Kunstteilmeniskus ausgebildet sein kann und auf seiner Außenfläche bzw. Oberfläche zusätzlich überhöht ist. Wird das erfindungsgemäße Implantat an die Stelle eines zuvor entfernten, defekten Meniskus bzw. Teilmeniskus zwischen die Kniegelenkflächen eines Patienten eingebracht, was mit besonderem Vorteil arthroskopisch durchgeführt werden kann, so wird das Implantat infolge der zusätzlichen Überhöhung durch die femoralen und tibialen Gelenkkondylen stärker nach außen an die Gelenkkapsel (Randleiste) gedrückt und dort gehalten als dies bei aus dem Stand der Technik bekannten Kunstmeniski bzw. Kunstteilmeniski der Fall ist. Zusätzliche Fixierungsschritte, insbesondere an die Gelenkkapsel bzw. Randleiste eines menschlichen oder tierischen Kniegelenks, sind in der Regel nicht mehr erforderlich.

In einer bevorzugten Ausführungsform sind der übererhöhte Formkörper und die zumindest eine zusätzliche Überhöhung einstückig ausgebildet. Beispielsweise können der überhöhte Formkörper und die zumindest eine zusätzliche Überhöhung monolithisch ausgebildet sein. Bevorzugt ist es, wenn die zumindest eine zusätzliche Überhöhung auf die Außenfläche des überhöhten Formkörpers angeformt ist. Der überhöhte Formkörper und die zumindest eine zusätzliche Überhöhung können kraftschlüssig, formschlüssig und/oder stoffschlüssig, bevorzugt stoffschlüssig, miteinander verbunden sein. Geeignete stoffschlüssige Verbindungen können beispielsweise auf Klebe-, Schweiß-, Löt-, Press-, Beschichtungs- und/oder Lyophilisationstechniken beruhen.

In einer weiteren bevorzugten Ausführungsform besitzt das Implantat einen Verbundaufbau aus dem überhöhten Formkörper und der zumindest einen zusätzlichen Überhöhung, insbesondere nach Art eines Komposits. Insbesondere können sich der überhöhte Formkörper und die zumindest eine zusätzliche Überhöhung entlang einer gemeinsamen Grenzfläche berühren. Vorzugsweise sind der überhöhte Formkörper und die zumindest eine zusätzliche Überhöhung entlang einer gemeinsamen Grenzfläche miteinander verbunden. Bezüglich geeigneter Verbindungsmöglichkeiten wird auf die im vorherigen Abschnitt beschriebenen Ausführungsformen Bezug genommen.

Die zumindest eine zusätzliche Überhöhung ist in einer weiteren Ausführungsform in Form einer Beschichtung auf der Außenfläche des überhöhten Formkörpers ausgebildet.

Die zumindest eine zusätzliche Überhöhung ist vorzugsweise auf zumindest einem Teil, insbesondere nur auf einem Teil, der Außenfläche des überhöhten Formkörpers ausgebildet. In der Regel ist die zumindest eine zusätzliche Überhöhung dabei vollflächig auf dem zumindest einen Teil der Außenfläche des überhöhten Formkörpers ausgebildet. Grundsätzlich kann die zumindest eine zusätzliche Überhöhung auf der femoralen Kondylen-Kontaktfläche, tibialen Kondylen-Kontaktfläche und/oder der Gelenkkapsel-Kontaktfläche des überhöhten Formkörpers ausgebildet sein. Bevorzugt ist die zumindest eine zusätzliche Überhöhung auf der femoralen Kondylen-Kontaktfläche und/oder tibialen Kondylen-Kontaktfläche des überhöhten Formkörpers ausgebildet. Ist die zumindest eine zusätzliche Überhöhung auf der Gelenkkapsel-Kontaktfläche des überhöhten Formkörpers ausgebildet, so ist es bevorzugt, wenn sie zusätzlich auch auf der femoralen und/oder tibialen Kondylen-Kontaktfläche des überhöhten Formkörpers ausgebildet ist, wodurch der überhöhte Formkörper mit besonderem Vorteil nach außen an die Gelenkkapsel bzw. Randleiste gedrückt wird.

In einer weiteren Ausführungsform weist das Implantat zwei zusätzliche Überhöhungen auf. Beispielsweise kann jeweils eine zusätzliche Überhöhung auf der femoralen Kondylen-Kontaktfläche und der tibialen Kondylen-Kontaktfläche des überhöhten Formkörpers ausgebildet sein. Alternativ kann auch jeweils eine zusätzliche Überhöhung auf der Außenfläche von Endbereichen des überhöhten Formkörpers ausgebildet sein. Auf Endbereichen des überhöhten Formkörpers ausgebildete zusätzliche Überhöhungen sind idealer Weise derart ausgebildet, dass sie mit der Außenflächenkontur der an den Endbereichen des überhöhten Formkörpers angrenzenden Gelenkanteile des Femur- und Tibia-Knochens korrespondieren können, so dass insbesondere im Bereich dieser Endbereiche eine feste Verankerung bzw. Verklemmung des Implantats möglich ist.

Das erfindungsgemäße Implantat, insbesondere der überhöhte Formkörper und/oder die zumindest eine zusätzliche Überhöhung, kann resorbierbar, teilresorbierbar oder nicht resorbierbar ausgebildet sein. Vorzugsweise ist das Implantat, insbesondere der überhöhte Formkörper und die zumindest eine zusätzliche Überhöhung, resorbierbar ausgebildet.

Grundsätzlich kann die zumindest eine zusätzliche Überhöhung aus dem gleichen Material gebildet sein wie der überhöhte Formkörper. Vorzugsweise ist die zumindest eine zusätzliche Überhöhung jedoch aus einem anderen Material gebildet als der überhöhte Formkörper. Bevorzugt ist die zumindest eine zusätzliche Überhöhung aus einem resorbierbaren Material, weiter bevorzugt aus einem anderen resorbierbaren, besonders bevorzugt aus einem schneller resorbierbaren, Material als der überhöhte Formkörper gebildet. Ist die zumindest eine zusätzliche Überhöhung resorbierbar bzw. schneller resorbierbar als der überhöhte Formkörper ausgebildet, so hat dies den Vorteil, dass es lediglich während der initialen Einheilungsphase zu einem vermehrten Anpressen des Implantats an die Gelenkkapsel bzw. Randleiste eines Patienten kommt. Im weiteren Verlauf der Einheilungsphase nimmt der Druck auf das Implantat infolge der Resorbierbarkeit bzw. schnelleren Resorbierbarkeit der zumindest einen zusätzlichen Überhöhung im Wesentlichen kontinuierlich ab. Idealerweise tritt an die Stelle der zumindest einen zusätzlichen Überhöhung nach der Resorption ein Meniskusregenerat, welches von Körperzellen des Patienten und/oder von gegebenenfalls im überhöhten Formkörper befindlichen Zellen aufgebaut wird.

Die zumindest eine zusätzliche Überhöhung besitzt in einer weiteren Ausführungsform einen schichtförmigen Aufbau. Vorzugsweise sind einzelne Schichten der zumindest einen zusätzlichen Überhöhung aus Materialien mit unterschiedlichen Resorptionsgeschwindigkeiten bzw. Resorptionszeiten gebildet. Vorzugsweise weisen einer Gelenkfläche, insbesondere einer femoralen und/oder tibialen Gelenkfläche, zugewandte Schichten der zumindest einen zusätzliche Überhöhung eine schnellere Resorptionsgeschwindigkeit auf als darunter liegende Schichten, insbesondere der zumindest einen zusätzlichen Überhöhung. Besonders bevorzugt ist es, wenn Schichten der zumindest einen zusätzlichen Überhöhung einen Resorptionsgradienten bilden. Vorzugsweise nimmt die Resorptionsgeschwindigkeit der Schichten von einer dem überhöhten Formkörper zugewandten Seite der zumindest einen zusätzlichen Überhöhung zu einer gegenüberliegenden, einer femoralen und/oder tibialen Gelenkfläche zugewandten Seite der zumindest einen zusätzlichen Überhöhung im Wesentlichen kontinuierlich zu. Mit anderen Worten ist es erfindungsgemäß besonders vorteilhaft, wenn die zumindest eine zusätzliche Überhöhung einen bezüglich der Resorptionszeit bzw. Resorptionsgeschwindigkeit schichtförmigen Aufbau besitzt. Dadurch ist es insbesondere möglich, dass Gelenkflächen zugewandte Schichten der zumindest einen zusätzlichen Überhöhung schneller resorbieren, wodurch die Dicke der zumindest einen zusätzlichen Überhöhung während der Einheilungsphase abnimmt, so dass das Implantat bzw. der überhöhte Formkörper schließlich unter physiologischen Bedingungen belastet wird.

In einer weiteren vorteilhaften Ausführungsform besitzt die zumindest eine zusätzliche Überhöhung eine im Wesentlichen glatte Außenfläche. Mit anderen Worten ist es erfindungsgemäß von Vorteil, wenn das Material der zumindest einen zusätzlichen Überhöhung gute Gleiteigenschaften besitzt. Des Weiteren kann es von Vorteil sein, wenn die zumindest eine zusätzliche Überhöhung eine reibungsarme Außenfläche besitzt. Mit anderen Worten ist es weiterhin von Vorteil, wenn das Material der zumindest einen zusätzlichen Überhöhung über gute tribologische Eigenschaften verfügt. Vorzugsweise ist die Außenfläche der zumindest einen zusätzlichen Überhöhung glatt und reibungsarm ausgebildet. Dies verbessert die Artikulation der zumindest einen zusätzlichen Überhöhung mit den Gelenkflächen des Kniegelenks. Erfindungsgemäß kann es weiterhin vorgesehen sein, dass die zumindest eine zusätzliche Überhöhung von einer reibungsarmen und insbesondere glatten Membran zum Gelenk hin abgedeckt ist.

In einer weiteren Ausführungsform ist die zumindest eine zusätzliche Überhöhung aus einem flexiblen und insbesondere weichen Material gebildet. Bevorzugt ist die zumindest eine zusätzliche Überhöhung aus einem flexibleren und insbesondere weicheren Material als der überhöhte Formkörper gebildet. Weiterhin kann die zumindest eine zusätzliche Überhöhung aus einem dehnbaren, insbesondere elastischen, Material gebildet sein. Vorzugsweise ist die zumindest eine zusätzliche Über-höhung aus einem flexibleren, insbesondere weicheren, Material gebildet als der überhöhte Formkörper.

Die zumindest eine zusätzliche Überhöhung ist in einer weiteren bevorzugten Ausführungsform porös ausgebildet. Das Implantat, insbesondere der überhöhte Formkörper und/oder die zumindest eine zusätzliche Überhöhung, weist vorzugsweise eine interkonnektierende Porosität auf. Erfindungsgemäß kann das Implantat, insbesondere der überhöhte Formkörper und/oder die zumindest eine zusätzliche Überhöhung, Poren mit einer Porengröße zwischen 50 und 500 µm, bevorzugt 100 und 350 µm, besonders bevorzugt 150 und 250 µm, aufweisen. Die zumindest eine zusätzliche Überhöhung ist vorzugsweise offenporös ausgebildet. Grundsätzlich kann die zumindest eine zusätzliche Überhöhung im Bereich der femoralen Kondylen-Kontaktfläche, tibialen Kondylen-Kontaktfläche und/oder der Gelenkkapsel-Kontaktfläche offenporös ausgebildet sein. Der überhöhte Formkörper ist im Bereich der Gelenkkapsel-Kontaktfläche, insbesondere nur im Bereich der Gelenkkapsel-Kontaktfläche, offenporös ausgebildet. Dies ist besonders vorteilhaft, da der natürliche Meniskus im Wesentlichen nur in seinem der Gelenkkapsel zugewandten Bereich (sogenannte Basis) Zellen aufweist. Die übrigen, insbesondere den femoralen und tibialen Gelenkflächen zugewandten Bereiche eines natürlichen Mensikus sind dagegen ausgesprochen zellarm. Dadurch können Körperzellen, insbesondere Stammzellen, Chondrozyten, Chondroblasten und/oder Fibroblasten, des Patienten ausgehend von der Gelenkkapsel das Implantat, insbesondere den überhöhten Formkörper und/oder die zumindest eine zusätzliche Überhöhung, besiedeln und durch den Aufbau von körpereigenem Bindegewebe, insbesondere Knorpelgewebe, zu einer dauerhaften Verankerung des Implantats, insbesondere des überhöhten Formkörpers und/oder der zumindest einen zusätzlichen Überhöhung, an der Gelenkkapsel des Patienten beitragen. Bevorzugt weist der überhöhte Formkörper und ggf. die zumindest eine zusätzliche Überhöhung an der Gelenkkapsel-Kontaktfläche offene Poren auf, die bis in eine Tiefe ausgebildet sind, die einem Drittel bis zwei Drittel der Breite des Implantats, insbesondere des überhöhten Formkörpers und/oder der zumindest einen zusätzlichen Überhöhung, entspricht. Sollen dagegen nur Körperzellen aus der Synovialflüssigkeit in die zumindest eine zusätzliche Überhöhung einsprossen, ist die zumindest eine zusätzliche Überhöhung vorzugsweise nur im Bereich der femoralen Kondylen-Kontaktfläche und/oder tibialen Kondylen-Kontaktfläche offenporös ausgebildet. In einer alternativen Ausführungsform ist die zumindest eine zusätzliche Überhöhung im Bereich der femoralen Kondylen-Kontaktfläche, tibialen Kondylen-Kontaktfläche und der Gelenkkapsel-Kontaktfläche offenporös ausgebildet. Auf diese Weise ist sowohl ein Einwachsen von Körperzellen aus der Synovialflüssigkeit, sogenannten Synovialzellen, als auch ein Einwachsen von anderen Körperzellen, insbesondere Stammzellen, Chondrozyten, Chondroblasten und/oder Fibroblasten, ausgehend von der Gelenkkapsel des Patienten in die zumindest eine zusätzliche Überhöhung möglich.

Der überhöhte Formkörper und ggf. die zumindest eine zusätzliche Überhöhung sind im Bereich der Gelenkkapsel-Kontaktfläche offenporös und im Bereich der femoralen Kondylen-Kontaktfläche und/oder tibialen Kondylen-Kontaktfläche geschlossen porig ausgebildet. Auf diese Weise wird beispielsweise ein Einwachsen von Körperzellen ausgehend von der Gelenkkapsel eines Patienten in den überhöhten Formkörper und ggf. die zumindest eine zusätzliche Überhöhung erleichtert. Die geschlossenen Poren im Bereich der femoralen Kondylen-Kontaktfläche und/oder tibialen Kondylen-Kontaktfläche des überhöhten Formkörpers und ggf. der zumindest einen zusätzlichen Überhöhung sorgen dagegen mit besonderem Vorteil für eine größere Flexibilität, insbesondere Dehnbarkeit, vorzugsweise Elastizität, wodurch sich wiederum eine bessere Anpassung an patientenspezifische Gelenkkonturen erzielen lässt.

Das Implantat, insbesondere der überhöhte Formkörper und/oder die zumindest eine zusätzliche Überhöhung, besitzt in einer weitergehenden Ausführungsform mit einem Material gefüllte Poren. Die Poren können dabei zumindest teilweise oder vollständig mit dem Material gefüllt sein. Bei dem Material selbst handelt es sich bevorzugt um ein resorbierbares und/oder viskoses, insbesondere hochviskoses, Material. Dadurch kann das Implantat, insbesondere der überhöhte Formkörper und/oder die zumindest eine zusätzliche Überhöhung, in einer unter physiologischen Gesichtspunkten ausreichenden Stabilität bereitgestellt werden. Ist das Füllmaterial resorbierbar und/oder viskos, insbesondere hochviskos, wird mit fortschreitender Resorption bzw. fortschreitendem Austritt des Materials aus den Poren Platz für einsprossende Körperzellen, insbesondere Stammzellen, Chondrozyten, Chondroblasten, Synovialzellen, Fibroblasten und Kombinationen davon, zur Verfügung gestellt. Bevorzugt weist das Implantat, insbesondere der überhöhte Formkörper und/oder die zumindest eine zusätzliche Überhöhung, im Bereich der Gelenkkapsel-Kontaktfläche, vorzugsweise lediglich im Bereich der Gelenkkapsel-Kontaktfläche, mit einem Material gefüllte Poren auf. Ist das Material, wie vorstehend beschrieben, resorbierbar oder viskos, insbesondere hochviskos, so kann mit besonderem Vorteil eine zelluläre Besiedelung ausgehend von der Gelenkkapsel eines Patienten in das Implantat, insbesondere den überhöhten Formkörper und/oder die zumindest eine zusätzliche Überhöhung, "erzwungen" werden.

Zum Gelenkknorpel zeigende Flächen des Implantats, insbesondere des überhöhten Formkörpers und/oder der zumindest einen zusätzlichen Überhöhung, können mit einer Membran, welche gegebenenfalls gute tribologische Eigenschaften aufweist, verschlossen bzw. bedeckt sein. Die Membran ermöglicht vorzugsweise die Diffusion von Gelenkflüssigkeit zur Ernährung von Zellen.

In einer weiteren Ausführungsform ist das Implantat, insbesondere der überhöhte Formkörper und/oder die zumindest eine zusätzliche Überhöhung, scheibenförmig, insbesondere in Form einer zylindrischen Scheibe, oder im Wesentlichen keilförmig ausgebildet. Insbesondere weist das Implantat, insbesondere der überhöhte Formkörper und/oder die zumindest eine zusätzliche Überhöhung, einen breiten Zentralbereich auf, der im Verhältnis dazu von zwei schmalen Endbereichen flankiert ist. Bevorzugt ist das Implantat, insbesondere der überhöhte Formkörper und/oder die zumindest eine zusätzliche Überhöhung, halbkreis-, C-förmig, sichel- oder halbmondförmig, ausgebildet. Das Implantat, insbesondere der überhöhte Formkörper und/oder die zumindest eine zusätzliche Überhöhung, kann weiterhin in Form eines kreisförmig verbreiterten Keils, insbesondere sichel- oder halbmondförmig verbreiterten Keils, ausgebildet sein. Liegt das Implantat, insbesondere der überhöhte Formkörper und/oder die zumindest eine zusätzliche Überhöhung, als Keil vor, weist dieser vorzugsweise einen Zentralbereich und einen Randbereich auf, wobei sich der Randbereich vorzugsweise über einen Winkel von mehr als 0° und ≤ 360° um den Zentralbereich erstreckt und der Randbereich eine größere Dicke besitzen kann als der Zentralbereich des Keils.

In einer weitergehenden Ausführungsform weist das Implantat, insbesondere der überhöhte Formkörper und/oder die zumindest eine zusätzliche Überhöhung, Verankerungselemente zur Verankerung des Implantats, insbesondere des überhöhten Formkörpers und/oder der zumindest einen zusätzliche Überhöhung, mit dem Kniegelenk eines Patienten auf. Die Verankerungselemente können grundsätzlich zur Verankerung des Implantats, insbesondere des überhöhten Formkörpers und/oder der zumindest einen zusätzlichen Überhöhung, mit dem femoralen Knochen, tibialen Knochen und/oder der Gelenkkkapsel vorgesehen sein. Grundsätzlich können die Verankerungselemente daher im Bereich der femoralen Kondylen-Kontaktfläche, der tibialen Kondylen-Kontaktfläche und/oder der Gelenkkapsel-Kontaktfläche des Implantats, insbesondere des überhöhten Formkörpers und/oder der zumindest einen zusätzlichen Überhöhung, ausgebildet sein. Vorzugsweise sind die Verankerungselemente im Bereich der Gelenkkapsel-Kontaktfläche ausgebildet. Geeignete Verankerungselemente sind aus der Gruppe bestehend aus Ankerhaken, insbesondere Widerhaken, Ankerpfeilen, Ankerstiften, Ankerschrauben und fadenförmigen Strukturen, insbesondere Fäden, ausgewählt. Kombinationen der vorstehend aufgeführten Verankerungselemente sind ebenfalls denkbar. Die Verankerungselemente sind in der Regel einstückig mit dem Implantat, insbesondere dem überhöhten Formkörper und/oder der zumindest einen zusätzlichen Überhöhung, ausgebildet. Beispielsweise können die Verankerungselemente vor der Implantation an das Implantat, insbesondere an den überhöhten Formkörper und/oder die zumindest eine zusätzliche Überhöhung, befestigt werden.

Die Verankerungselemente selbst bestehen vorzugsweise aus einem resorbierbaren Material. Des Weiteren ist es von Vorteil, wenn die Verankerungselemente aus einem Material gebildet sind, welches sich beispielsweise in die Gelenkkapsel bzw. Randleiste eines Patienten einpressen lässt, wodurch sich aufgrund des dadurch erzielbaren Form- und/oder Stoffschlusses eine bessere Verankerung des erfindungsgemäßen Implantats, insbesondere des überhöhten Formkörpers und/oder der zumindest einen zusätzlichen Überhöhung, erzielen lässt. Ein weiterer Vorteil besteht darin, dass mittels Verankerungselementen gezielte und kontrollierbare Verletzungs- und Entzündungsreize verursacht werden können, wodurch ein Einwachsen des Implantats, insbesondere des überhöhten Formkörpers und/oder der zumindest einen zusätzlichen Überhöhung im Kniegelenkbereich eines Patienten begünstigt wird. Handelt es sich bei den Verankerungselementen um Fäden, so kann es sich bei diesen Fäden um in das Implantat, insbesondere den überhöhten Formkörper, fest eingearbeitete und aus diesem austretende Fäden handeln. Mit Hilfe von Fäden als Verankerungselementen kann das Implantat insbesondere an der Gelenkkapsel bzw. Randleiste eines Patienten, gegebenenfalls mit Hilfe eines geeigneten Instrumentariums, vernäht werden. Die Verwendung von Fäden als Verankerungselemente hat außerdem den Vorteil, dass diese nach einer gewissen Zeit in das Bindegewebe eines Patienten integriert werden können und auf diese Weise ebenso das Einwachsen des Implantats, insbesondere des überhöhten Formkörpers und/oder der zumindest einen zusätzlichen Überhöhung, an der Gelenkkapsel eines Patienten begünstigen.

In einer weiteren Ausführungsform weist das Implantat, insbesondere der überhöhte Formkörper und/oder die zumindest eine zusätzliche Überhöhung, nur im Bereich der femoralen Kondylen-Kontaktfläche und/oder tibialen Kondylen-Kontaktfläche Verankerungselemente, insbesondere wie sie im vorherigen Abschnitt beschrieben sind, auf. Als besonders bevorzugte Verankerungselemente werden in diesem Zusammenhang Fäden genannt. Diese können mittels entsprechender Instrumente durch einen menschlichen oder tierischen Knochen gezogen und an diesem befestigt werden. Alternativ können die Fäden an ihrem freien Ende mittels eines oder mehrerer anderer Verankerungselemente verbunden werden. Die mit einem bzw. mehreren anderen Verankerungselementen verbundenen Fäden können mit Hilfe eines geeigneten Instrumentes im Knochen fixiert und die Fäden gegebenenfalls zum Erreichen einer gewünschten Vorspannung angezogen werden. Erfindungsgemäß ist es aber ebenso möglich, dass besagte Verankerungselemente widerhakenförmige Strukturen aufweisen, wodurch sie sich beispielsweise im Gewebe der Gelenkkapsel eines Patienten verankern lassen und auf diese Weise das Implantat, insbesondere den überhöhten Formkörper und/oder die zumindest eine zusätzliche Überhöhung, im Bereich der femoralen Kondylen-Kontaktfläche und/oder tibialen Kondylen-Kontaktfläche stabilisieren.

Wie bereits erwähnt, eignet sich das erfindungsgemäße Implantat auch zur Rekonstruktion von Meniskusteildefekten. Mit anderen Worten kann das Implantat als Teilkunstmeniskus ausgebildet sein. Hierbei können insbesondere die in den vorherigen Ausführungsformen beschriebenen Verankerungselemente zum Einsatz kommen. Insbesondere kann es erfindungsgemäß vorgesehen sein, dass ein als Teilkunstmeniskus ausgebildetes Implantat an seinen Enden, an denen es den natürlichen und im Gelenk belassenen Meniskus berührt, Verankerungselemente aufweist. Die Verankerungselemente können in den natürlichen Restmeniskus hineingedrückt werden und sich beispielsweise mittels Formschluss in fester Weise mit dem natürlichen Restmeniskus verbinden. Die Verankerungselemente können weiterhin entweder fest mit dem Implantat verbunden sein oder aber unmittelbar vor der Operation mit dem Implantat verbunden und/oder in dem natürlichen Restmeniskus verankert werden. Alternativ oder in Kombination dazu kann es erfindungsgemäß sinnvoll sein, das als Teilkunstmeniskus ausgebildete Implantat mit einem natürlichen Restmeniskus, beispielsweise mittels eines Fibrinklebers und/oder eines auf Cyanoacrylat-Monomeren basierenden Gewebeklebers, zu verkleben. Um die Anhaftung des Klebers an den Teilkunstmeniskus zu verbessern, können die dem natürlichen Meniskus anliegenden Seiten des als Teilkunstmeniskus ausgebildeten Implantats aufgeraut oder mit einer Beschichtung versehen werden, welche sich besonders gut mit dem verwendeten Kleber verbindet. Es ist auch möglich, aus den dem natürlichen Restmeniskus anliegenden Flächen des Implantats Fäden herausragen zu lassen, an welche der verwendete Kleber besonders gut haftet. Bevorzugt werden hierbei Fäden verwendet, welche sich aufgrund ihrer Materialeigenschaften oder aufgrund einer Oberflächenbeschichtung besonders gut mit einem Kleber verbinden lassen.

Oftmals finden sich Meniskusdefekte auch an den Endstellen eines natürlichen Meniskus. In diesen Fällen ist es von Vorteil, wenn ein als Teilkunstmeniskus ausgebildetes Implantat an der dem natürlichen Restmeniskus anliegenden Seite Verankerungselemente aufweist.

In einer weiteren Ausführungsform weist das Implantat Verankerungselemente für eine Verankerung zwischen dem überhöhten Formkörper und der zumindest einen zusätzlichen Überhöhung auf.

In einer weiteren Ausführungsform weist das Implantat, insbesondere der überhöhte Formkörper einen Abstandshalter bzw. Spacer auf, der für eine gegenseitige Beabstandung der femoralen und tibialen Gelenkflächen vorgesehen ist. Besonders bevorzugt ist die zumindest eine zusätzliche Überhöhung ferner als Abstandshalter bzw. Spacer ausgebildet, insbesondere zur gegenseitigen Beabstandung der femoralen und tibialen Gelenkflächen. Der Spacer kann sich bis zu dem Notchbereich des Kniegelenks erstrecken oder sogar in den Notchbereich (Dach zwischen den Femurkondylen bzw. Fossa interkondylaris) des Kniegelenks hineinragen. Da der Spacer in der Regel zwischen den Gelenkflächen des Femur-Knochens und Tibia-Knochens liegt und vorzugsweise der Form der Femur-Kondylen und der Tibia-Kondylen angepasst ist und somit stabil zwischen diesen Gelenkflächen gelegt werden kann, bewirkt der Spacer eine zusätzliche Stabilisierung des erfindungsgemäßen Implantats im Gelenk. Erfindungsgemäß ist es besonders bevorzugt, wenn der Spacer eine kürzere Resorptionszeit als der überhöhte Formkörper (Kunstmeniskus bzw. Kunstteilmeniskus) besitzt. Der Spacer und der überhöhte Formkörper können einstückig oder physikalisch getrennt voneinander ausgebildet sein. Sind der Spacer und der überhöhte Formkörper nicht fest miteinander verbunden, so kann sich der Spacer beispielsweise gegen die Gelenkflächen der femoralen und tibialen Tibia-Kondylen sowie gegen den überhöhten Formkörper abstützen, wodurch das Implantat, insbesondere der überhöhte Formkörper und/oder die zumindest eine zusätzliche Überhöhung, nach außen an die Gelenkkapsel bzw. Randleiste gedrückt wird. Auf diese Weise lässt sich die Verankerung im Bereich der Gelenkkapsel des Kniegelenkes eines Patienten noch weiter verbessern.

Das Implantat, insbesondere der überhöhte Formkörper und/oder die zumindest eine zusätzliche Überhöhung, kann grundsätzlich alle biokompatiblen Materialien aufweisen bzw. aus allen biokompatiblen Materialien gebildet sein. Die Materialien können resorbierbar, teilresorbierbar oder nicht resorbierbar sein. Bevorzugt weist das Implantat, insbesondere der überhöhte Formkörper und/oder die zumindest eine zusätzliche Überhöhung, resorbierbare Materialien auf bzw. ist aus solchen Materialien gebildet. Bevorzugt handelt es sich bei den in Frage kommenden Materialien um natürlich vorkommende Polymere, sogenannte Biopolymere, und/oder synthetische Polymere. Generell sind Polymere bevorzugt, welche bezüglich ihrer elastische Eigenschaften vergleichbar sind mit einem natürlichen Gelenkknorpel- und/oder Meniskusgewebe. Geeignete Polymere sind bevorzugt aus der Gruppe bestehend aus Proteine, Polysaccharide, Polyhydroxyalkanoate und Kombinationen davon ausgewählt. Geeignete Polysaccharide sind insbesondere aus der Gruppe bestehend aus Glykosaminoglykane, Cellulosederivate und Kombinationen davon ausgewählt. Bei den Cellulosederivaten kann es insbesondere um Alkylcellulosen, Hydroxyalkylcellulosen, Carboxyalkylcellulosen oder Kombinationen davon handeln. Bevorzugte Polyhydroxyalkanoate sind von α-, β- und/oder γ-Hydroxysäuren abgeleitet. Geeignete Biopolymere und/oder synthetische Polymere können beispielsweise aus der Gruppe bestehend aus Albumin, Gelatine, Collagen, Elastin, Retikulin, Chondroitin-4-Sulfat, Chondroitin-6-Sulfat, Keratansulfat, Dermatansulfat, Heparinsulfat, Hyaluronsäure, Chitosan, Algininsäure, Methylcellulose, Hydroxymethylcellulose, Hydroxyethylcellulose, Hydroxypropylcellulose, Hydroxybutylcellulose, Carboxymethylcellulose, Polylactid, Polyglykolid, Poly-ε-caprolacton, Polytrimethylencarbonat, Poly-para-dioxanon, Poly-3-hydroxybutyrat, Poly-4-hydroxybutyrat, Polyvinylalkohol, Polyurethane, Derivate davon, Analoga davon, Copolymere davon und Kombinationen davon ausgewählt sein. Geeignete Polyurethane können insbesondere aus der Gruppe bestehend aus aromatische Polycarbonaturethane, aliphatische Polycarbonaturethane, Silikon-Polycarbonaturethane, Silikon-Polyetherurethane, Derivate davon, Analoga davon und Kombinationen davon ausgewählt sein.

In einer weiteren möglichen Ausführungsform ist das Implantat, insbesondere der überhöhte Formkörper und/oder die zumindest eine zusätzliche Überhöhung, aus einem Transplantationsmaterial, insbesondere alloplastischem und/oder autologem Transplantationsmaterial, gebildet. Bei dem Transplantationsmaterial handelt es sich vorzugsweise um ein Binde- und/oder Stützgewebe, insbesondere Knorpelgewebe.

In einer weiteren bevorzugten Ausführungsform weist das Implantat, insbesondere der überhöhte Formkörper und/oder die zumindest eine zusätzliche Überhöhung, einen Anteil eines faserförmigen Materials auf. Durch das faserförmige Material lässt sich mit besonderem Vorteil die mechanische Stabilität des Implantats, insbesondere des überhöhten Formkörpers und/oder der zumindest einen zusätzlichen Überhöhung, verbessern. Geeignete faserförmige Materialien können beispielsweise aus der Gruppe bestehend aus Polyglykolid, Polylactid, Polytrimethylencarbonat, Poly-ε-Caprolacton, Poly-para-Dioxanon, Cellulose bzw. Cellulosederivate, Hyaluronsäure und Kombinationen davon ausgewählt sein. Vorzugsweise weist das Implantat, insbesondere der überhöhte Formkörper und/oder die zumindest eine zusätzliche Überhöhung, eine inhomogene Faserausrichtung auf. Beispielsweise können Fasern des faserförmigen Materials im Falle eines halbkreis-, sichel- oder halbmondförmig ausgebildeten Implantats in radiärer Richtung ausgerichtet sein. Weist das Implantat einen Abstandshalter bzw. Spacer auf, so können Fasern des faserförmigen Materials in Längs- und/oder Querrichtung des Abstandshalters angeordnet sein. Bevorzugt weist das Implantat, insbesondere der überhöhte Formkörper und/oder die zumindest eine zusätzliche Überhöhung, einen Anteil des faserförmigen Materials zwischen 2 und 80 Gew.-%, insbesondere 10 und 30 Gew.-%, vorzugsweise 15 und 25 Gew.-%, auf, bezogen auf das Gesamtgewicht des Implantats.

Das Implantat, insbesondere der überhöhte Formkörper und/oder die zumindest eine zusätzliche Überhöhung, ist in einer weiteren Ausführungsform mit Zellen, insbesondere Körperzellen, vorzugsweise autologen Körperzellen, inokuliert bzw. beimpft. Die Zellen sind vorzugsweise aus der Gruppe bestehend aus Chondrozyten, Chondroblasten, Synovialzellen, Fibroblasten, Vorläuferzellen davon, Stammzellen davon und Kombinationen davon ausgewählt. Durch die Anwesenheit von Körperzellen im Implantat, insbesondere in dem überhöhten Formkörper und/oder der zumindest einen zusätzlichen Überhöhung, lässt sich der Aufbau eines Meniskusregenerats beschleunigen. Die Zellen selbst können beispielsweise aus dem Knochenmark eines Patienten stammen oder bei einer Operation dem Körper eines Patienten entnommen worden sein.

Weiterhin kann das Implantat, insbesondere der überhöhte Formkörper und/oder die zumindest eine zusätzliche Überhöhung, Wirkstoffe, insbesondere biologische Wirkstoffe, enthalten. Die Wirkstoffe können aus der Gruppe bestehend aus Wachstumsfaktoren, Rekrutierungsfaktoren, Differenzierungsfaktoren, Adhäsionsfaktoren und Kombinationen davon ausgewählt sein. Mittels derartiger Wirkstoffe kann eine schnellere Zellbesiedelung in das Implantat und insbesondere eine schnellere Differenzierung von in das Implantat einsprossenden Zellen in Bindegewebe, insbesondere Knorpelgewebe, erzielt werden. Bei den Wirkstoffen kann es sich beispielsweise um chrondroinduktive, fibroblasteninduktive, fibroblastenkonduktive und/oder die Angiogenese stimulierende Wirkstoffe handeln. Bevorzugt weist das Implantat, insbesondere der überhöhte Formkörper und/oder die zumindest eine zusätzliche Überhöhung, im Bereich der Gelenkkapsel-Kontaktfläche fibroblasteninduktive, fibroblastenkonduktive und/oder die Angiogenese stimulierende Wirkstoffe auf, wohingegen andere Bereiche, vor allem die femorale Kondylen-Kontaktfläche und/oder die tibiale Kondylen-Kontaktfläche, des Implantats, insbesondere des überhöhten Formkörpers und/oder der zumindest einen zusätzlichen Überhöhung, bevorzugt chondroinduktive Wirkstoffe aufweisen. Weiterhin können geeignete Wirkstoffe auch aus der Gruppe bestehend aus antimikrobielle Wirkstoffe, desinfizierende Wirkstoffe, entzündungshemmende Wirkstoffe, schmerzlindernde Wirkstoffe und Kombinationen davon ausgewählt sein. Erfindungsgemäß kann es weiterhin vorgesehen sein, dass die Wirkstoffe inhomogen im Implantat, insbesondere im überhöhten Formkörper und/oder der zumindest einen zusätzlichen Überhöhung, verteilt sind. Beispielsweise können die Wirkstoffe im Bereich der Gelenkkapsel-Kontaktfläche des Implantats in höheren Konzentrationen vorliegen als in anderen Bereichen des Implantats. Erfindungsgemäß ist es weiterhin bevorzugt, wenn beispielweise die Konzentration von Wachstumsfaktoren, Rekrutierungsfaktoren, Differenzierungsfaktoren, Adhäsionsfaktoren und/oder Kombinationen davon im Bereich der Gelenkkapsel-Kontaktfläche des Implantats höher ist als in anderen Bereichen. Dadurch lässt sich das Einwachsen von Körperzellen ausgehend von der Gelenkkapsel beschleunigen. Insbesondere können erfindungsgemäß gegebenenfalls vorgesehene Verankerungselemente des Implantats, insbesondere des überhöhten Formkörpers und/oder der zumindest einen zusätzlichen Überhöhung, Wirkstoffe, wie sie vorstehend beschrieben sind, enthalten oder aus Materialien mit einer entsprechenden Wirkung gebildet sein.

Die Erfindung betrifft weiterhin ein chirurgisches Set zur Rekonstruktion von Meniskusdefekten oder Meniskusteildefekten, wobei das chirurgische Set zumindest ein Implantat der vorliegenden Erfindung umfasst. Des Weiteren kann das Set eine Komponente, ausgewählt aus der Gruppe bestehend aus Abstandshalter bzw. Spacer zur gegenseitigen Beabstandung einer femoralen Kondyle und einer tibialen Kondyle und/oder einen Gewebekleber umfassen. Bezüglich weiterer Merkmale und Einzelheiten wird vollständig auf die bisherige Beschreibung Bezug genommen.

Ein weiterer Aspekt der vorliegenden Offenbarung betrifft ein Verfahren zur Rekonstruktion von Meniskusdefekten oder Meniskusteildefekten, wobei ein Implantat gemäß der vorliegenden Erfindung nach Entfernung eines natürlichen Meniskus oder Teilmeniskus zwischen eine tibiale Kondyle und eine mit der tibialen Kondyle artikulierende femorale Kondyle eines natürlichen Kniegelenks implantiert wird. Es kann dabei vorgesehen sein, dass die tibiale Kondyle und/oder die femorale Kondyle vor dem Implantieren des Implantats einer Mikrofrakturierung unterworfen werden. Des Weiteren kann es vorgesehen sein, dass das Implantat vor dem Implantieren mit Zellen, bevorzugt Körperzellen, insbesondere autologen Körperzellen, vorzugsweise ausgewählt aus der Gruppe bestehend aus Chondrozyten, Chondroblasten, Synoviozyten, Fibroblasten, Vorläuferzellen davon, Stammzellen davon und Kombinationen davon, inokuliert wird.

Weitere Merkmale und Vorteile der Erfindung ergeben sich aus der nachfolgenden Beschreibung bevorzugter Ausführungsformen anhand der Figurenbeschreibungen, der Figuren selbst sowie der Unteransprüche. Hierbei können die Merkmale jeweils für sich alleine oder in Kombination miteinander verwirklicht sein.

In den Figuren zeigen schematisch:
- Figur 1:: ein natürliches Kniegelenk,
- Figur 2:: eine Draufsicht auf einen aus dem Stand der Technik bekannten Kunstmeniskus,
- Figur 3:: eine geschnittene Schrägansicht eines aus dem Stand der Technik bekannten Kunstmeniskus,
- Figur 4:: eine Querschnittsansicht eines nicht erfindungsgemäßen Implantats mit Verankerungselementen,
- Figur 5:: eine geschnittene Schrägansicht eines nicht erfindungsgemäßen Implantats mit Verankerungselementen,
- Figur 6:: eine Querschnittsansicht eines erfindungsgemäßen Implantats mit zwei zusätzlichen Überhöhungen,
- Figur 7:: eine Querschnittsansicht eines erfindungsgemäßen Implantats mit einer zu der femoralen Gelenkfläche hin zeigenden zusätzlichen Überhöhung,
- Figur 8:: eine Querschnittsansicht eines erfindungsgemäßen Implantats mit einer zu der tibialen Gelenkfläche hin zeigenden zusätzlichen Überhöhung.

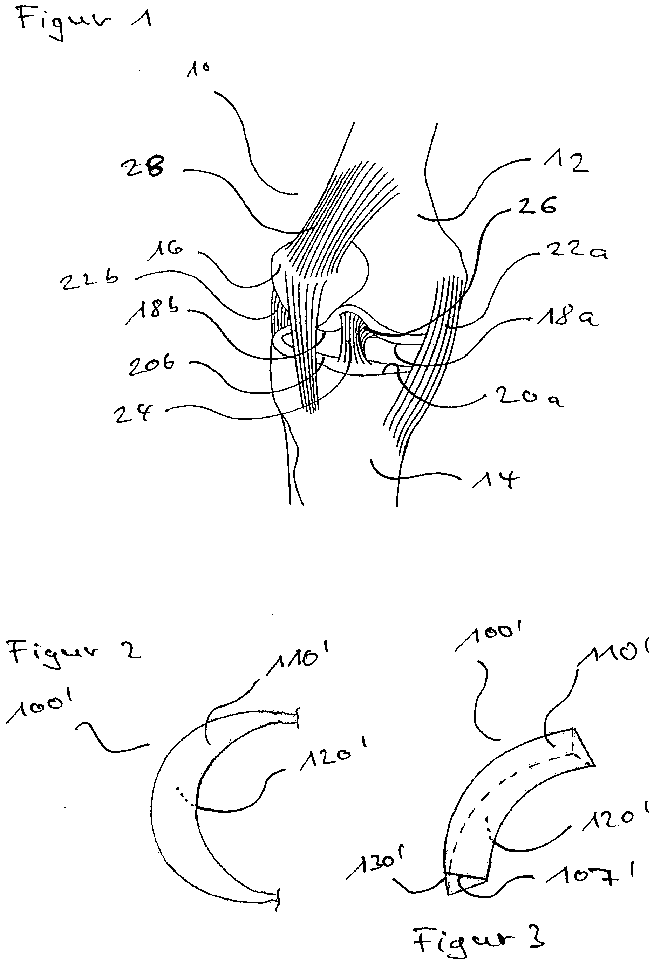

Figur 1 zeigt ein natürliches Kniegelenk 10. Am Aufbau des Kniegelenks sind als knöcherne Gelenkpartner der Oberschenkelknochen 12 (Femur), das Schienbein 14 (Tibia) und die Kniescheibe 16 (Patella) beteiligt. Der Oberschenkelknochen endet kniewärts (distal) mit zwei relativ breiten, leicht nach außen gekrümmten (konvexen) Gelenkknorren 18a; b (Condylus lateralis femoris 18a und Condylus medialis femoris 18b). Das obere Ende des Schienbeins 14 endet ebenfalls in zwei, allerdings leicht nach innen gekrümmten (konkaven) Gelenkknorren 20a;b (Condylus lateralis tibiae 20a und Condylus medialis tibiae 20b). Zwischen den vorstehend beschriebenen Gelenkflächen liegen der mediale Meniskus und der laterale Meniskus als paarige, halbmondförmige Faserknorpelscheiben zum Ausgleich der Inkongruenz der artikulierenden Knochen (die Meniski sind in der Figur 1 nicht dargestellt). Von dem ebenfalls am Aufbau des Kniegelenks beteiligten Bandapparat sind in der Figur 1 die Seitenbänder 22a;b (Ligamentum collaterale fibulare 22a und Ligamentum collaterale tibiale 22b), das vordere Kreuzband 24 (Ligamentum cruciatum anterius), das hintere Kreuzband 26 (Ligamentum posterior) sowie die Kniescheibensehne 28 (Ligamentum patellae) dargestellt.

Figur 2 zeigt eine Draufsicht auf einen aus dem Stand der Technik bekannten Kunstmeniskus 100'. Der Kunstmeniskus besitzt eine femorale Kondylen-Kontaktfläche 110', eine tibiale Kondylen-Kontaktfläche 120' sowie eine Gelenkkapsel- bzw. Randleisten-Kontaktfläche 130' (Basis). Der Kunstmeniskus 100' ist sichel- bzw. halbmondförmig ausgebildet. Der in Figur 2 dargestellte Kunstmeniskus 100' eignet sich für eine vollständige Meniskusrekonstruktion. Augrund der in Figur 2 beispielhaft dargestellten offenen Sichelform eignet sich der Kunstmeniskus 100' insbesondere für eine Rekonstruktion des medialen Meniskus.

Figur 3 zeigt schematisch eine geschnittene Schrägansicht des in Figur 2 dargestellten Kunstmeniskus 100'. Aus der Figur 3 wird deutlich, dass der Kunstmeniskus 100' an sich bereits überhöht ist bzw. eine Überhöhung 107' aufweist.

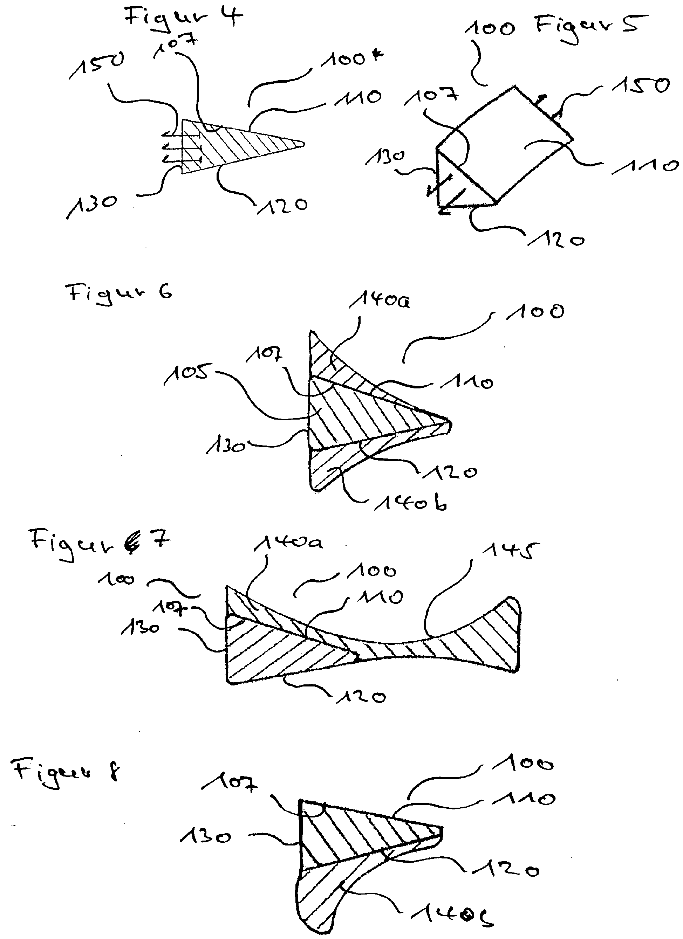

Figur 4 zeigt die Querschnittsfläche eines nicht erfindungsgemäßen Implantats 100. Das Implantat 100 basiert auf einem als Kunstmeniskus oder Kunstteilmeniskus ausgebildeten überhöhten Formkörper 105 (Formkörper 105 besitzt an sich eine Überhöhung 107). Der überhöhte Formkörper 105 besitzt eine femorale Kondylen-Kontaktfläche 110, eine tibiale Kondylen-Kontaktfläche 120 sowie eine Gelenkkapsel-Kontaktfläche 130 (Basis). Der überhöhte Formkörper 105 besitzt zusätzlich Verankerungselemente 150. Die Verankerungselemente 150 können, wie in Figur 4 beispielhaft dargestellt, pfeilförmig ausgebildet sein. Die Verankerungselemente 150 sind integral mit dem überhöhte Formkörper 105 verbunden und treten aus dessen Gelenkkapsel-Kontaktfläche 130 heraus. Mittels der Verankerungselemente 150 lässt sich das Implantat 100 an die Gelenkkapsel bzw. Randleiste eines menschlichen oder tierischen Kniegelenks fixieren. Dabei sorgen die Verankerungselemente 150 beim Verankern in der Gelenkkapsel mit besonderem Vorteil für gezielte und kontrollierte Entzündungsreize, welche die Verankerung des Implantats 100, insbesondere das Einwachsen von Körperzellen in das Implantat 100 und gegebenenfalls den Aufbau von Binde- und/oder Stützgewebe, insbesondere Knorpelgewebe, beschleunigen können.

Figur 5 zeigt schematisch ein weiteres nicht erfindungsgemäßes Implantat 100. Das Implantat 100 weist einen als Kunstteilmeniskus ausgebildeten überhöhten Formkörper 105 auf (Formkörper 105 besitzt an sich eine Überhöhung 107). Der überhöhte Formkörper 105 besitzt ebenfalls eine femorale Kondylen-Kontaktfläche 110, eine tibiale Kondylen-Kontaktfläche 120 sowie eine Gelenkkapsel-Kontaktfläche 130 (Basis). Der überhöhte Formkörper 105 ist im Wesentlichen keilförmig ausgebildet und weist an den Enden des Keils, welche jeweils zur Angrenzung an den natürlichen Restmeniskus vorgesehen sind, Verankerungselemente 150 auf. Bezüglich weiterer Merkmale und Vorteile zu den Verankerungselementen 150 wird auf die Beschreibung zu Figur 5 verwiesen.

Figur 6 zeigt die Querschnittsfläche einer bevorzugten Ausführungsform für ein erfindungsgemäßes Implantats 100. Das Implantat basiert auf einem überhöhten Formkörper 105 (Formkörper 105 weist an sich eine Überhöhung 107 auf). Der überhöhte Formkörper 105 weist eine femorale Kondylen-Kontaktfläche 110, eine tibiale Kondylen-Kontaktfläche 120 sowie eine Gelenkkapsel-Kontaktfläche 130 (Basis) auf. Der überhöhte Formkörper 105 kann dabei insbesondere als Kunstmeniskus oder Kunstteilmeniskus, wie er beispielsweise in Figur 2 dargestellt ist, ausgebildet sein. Das Implantat 100 weist zwei zusätzliche Überhöhungen 140a;b auf. Diese sind als zusätzliche Überhöhungen auf dem an sich bereits überhöhten Formkörper ausgebildet, und zwar auf der femoralen Kondylen-Kontaktfläche 110 und tibialen Kondylen-Kontaktfläche 120 des überhöhten Formkörpers 105. Die zusätzliche Überhöhung 140a ist auf ihrer der Gelenkfläche zugewandten Seite konkav ausgebildet, damit sie mit einer femoralen Kondyle kooperieren kann. Die zusätzliche Überhöhung 140b ist auf ihrer der Gelenkfläche zugewandten Seite dagegen (leicht) konvex ausgebildet, damit sie mit einer tibialen Kondyle kooperieren kann.

Figur 7 zeigt eine weitere bevorzugte Ausführungsform für ein erfindungsgemäßes Implantat 100. Das Implantat 100 basiert, wie das in Figur 6 dargestellte Implantat, auf einem überhöhten Formkörper 105, der nach Art eines Kunstmeniskus oder Kunstteilmeniskus ausgebildet sein kann. Der überhöhte Formkörper 105 weist weiterhin auch eine femorale Kondylen-Kontaktfläche 110, eine tibiale Kondylen-Kontaktfläche 120 sowie eine Gelenkkapsel-Kontaktfläche 130 (Basis) auf. Auf der Außenfläche des überhöhten Formkörpers 105 ist zusätzliche eine Überhöhung 140a auf der femoralen Kondylen-Kontaktfläche ausgebildet. Die zusätzliche Überhöhung 140a weist gleichzeitig einen Spacerbereich 145 auf, der zwischen die femoralen und tibialen Gelenkflächen hineinragt und sich bis zum Notchbereich eines Kniegelenks erstrecken kann. Die zusätzliche Überhöhung 140a ist auf ihrer der femoralen Gelenkfläche zugewandten Seite (leicht) konkav ausgebildet, um mit einer femorale Kondyle kooperieren zu können.

Figur 8 zeigt schematisch eine weitere bevorzugte Ausführungsform für ein erfindungsgemäßes Implantat 100. Das Implantat 100 basiert, wie das in Figur 7 dargestellte Implantat, auf einem überhöhten Formkörper 105, der nach Art eines Kunstmeniskus oder Kunstteilmeniskus ausgebildet sein kann. Der überhöhte Formkörper 105 weist ebenfalls eine femorale Kondylen-Kontaktfläche 110, eine tibiale Kondylen-Kontaktfläche 120 sowie eine Gelenkkapsel-Kontaktfläche 130 (Basis) auf. Auf der Außenfläche des überhöhten Formkörpers 105 ist zusätzliche eine Überhöhung 140b auf der tibialen Kondylen-Kontaktfläche ausgebildet. Die Überhöhung 140b ist auf der der tibialen Kondylen-Kontaktfläche zugewandten Seite (leicht) konvex ausgebildet, um die Kooperation mit einer tibialen Kondyle zu ermöglichen. Bezüglich weiterer Merkmale und Einzelheiten wird auf die Beschreibung zur Figur 7 Bezug genommen.

## Patentansprüche

1. Implantat (100) zur Rekonstruktion von Meniskusdefekten oder Meniskusteildefekten, umfassend einen überhöhten Formkörper (105) mit einer femoralen Kondylen-Kontaktfläche (110), einer tibialen Kondylen-Kontaktfläche (120) und einer Gelenkkapsel-Kontaktfläche (130), insbesondere nach Art eines Kunstmeniskus oder Kunstteilmeniskus mit einer im Wesentlichen einem natürlichen Meniskus bzw. Teilmeniskus gleichenden Außenflächenkontur, wobei auf der Außenfläche des überhöhten Formkörpers (105) zumindest eine zusätzliche Überhöhung (140a;b) ausgebildet ist, **dadurch gekennzeichnet, dass** der überhöhte Formkörper (105) im Bereich der Gelenkkapsel-Kontaktfläche (130) offenporös und im Bereich der femoralen Kondylen-Kontaktfläche (110) und/oder tibialen Kondylen-Kontaktfläche (120) geschlossen porig ausgebildet ist.

2. Implantat (100) nach Anspruch 1, **dadurch gekennzeichnet, dass** das Implantat (100) einen Verbundaufbau aus dem überhöhten Formkörper (105) und der zumindest einen zusätzlichen Überhöhung (140a;b), insbesondere nach Art eines Komposits, besitzt.

3. Implantat (100) nach Anspruch 1 oder 2, **dadurch gekennzeichnet, dass** die zumindest eine zusätzliche Überhöhung (140a;b) auf der femoralen Kondylen-Kontaktfläche (110), tibialen Kondylen-Kontaktfläche (120) und/oder Gelenkkapsel-Kontaktfläche (130), vorzugsweise auf der femoralen Kondylen-Kontaktfläche (110) und/oder tibialen Kondylen-Kontaktfläche (120), des überhöhten Formkörpers (105) ausgebildet ist.

4. Implantat (100) nach einem der vorhergehenden Ansprüche, **dadurch gekennzeichnet, dass** die zumindest eine zusätzliche Überhöhung (140a;b) aus einem anderen Material gebildet ist als der überhöhte Formkörper (105).

5. Implantat (100) nach einem der vorhergehenden Ansprüche, **dadurch gekennzeichnet, dass** die zumindest eine zusätzliche Überhöhung (140a;b) aus einem resorbierbaren Material, insbesondere schneller resorbierbaren Material als der überhöhte Formkörper (105), gebildet ist.

6. Implantat (100) nach einem der vorhergehenden Ansprüche, **dadurch gekennzeichnet, dass** Schichten der zumindest einen zusätzlichen Überhöhung (140a;b) einen Resorptionsgradienten bilden, wobei vorzugsweise die Resorptionsgeschwindigkeit der Schichten von einer dem überhöhten Formkörper (105) zugewandten Seite der zumindest einen zusätzlichen Überhöhung (140a;b) zu einer gegenüberliegenden Seite der zumindest einen zusätzlichen Überhöhung (140a;b) im Wesentlichen kontinuierlich zunimmt.

7. Implantat (100) nach einem der vorhergehenden Ansprüche, **dadurch gekennzeichnet, dass** die zumindest eine zusätzliche Überhöhung (140a;b) aus einem flexibleren, insbesondere weicheren, bevorzugt elastischeren, Material gebildet ist als der überhöhte Formkörper (105).

8. Implantat (100) nach einem der vorhergehenden Ansprüche, **dadurch gekennzeichnet, dass** die zumindest eine zusätzliche Überhöhung (140a;b), porös, insbesondere offenporös, ausgebildet ist, wobei der überhöhte Formkörper (105) im Bereich seiner Gelenkkapsel-Kontaktfläche (130) offenporös ausgebildet ist.

9. Implantat (100) nach einem der vorhergehenden Ansprüche, **dadurch gekennzeichnet, dass** die zumindest eine zusätzliche Überhöhung (140a;b) geschlossene Poren enthält.

10. Implantat (100) nach einem der vorhergehenden Ansprüche, **dadurch gekennzeichnet, dass** offene Poren des Implantats (100), insbesondere des überhöhten Formkörpers (105) und/oder der zumindest einen zusätzlichen Überhöhung (140a;b), mit einem resorbierbaren und/oder viskosen Material zumindest teilweise gefüllt sind.

11. Implantat (100) nach einem der vorhergehenden Ansprüche, **dadurch gekennzeichnet, dass** das Implantat (100), insbesondere der überhöhte Formkörper (105) und/oder die zumindest eine zusätzliche Überhöhung (140a;b), scheibenförmig, halbkreisförmig, sichelförmig oder halbmondförmig ausgebildet ist.

12. Implantat (100) nach einem der vorhergehenden Ansprüche, **dadurch gekennzeichnet, dass** das Implantat (100), insbesondere der überhöhte Formkörper (105) und/oder die zumindest eine zusätzliche Überhöhung (140a;b), Verankerungselemente (150), vorzugsweise ausgewählt aus der Gruppe bestehend aus Ankerhaken, Ankerpfeilen, Ankerstiften, Ankerschrauben, fadenförmigen Strukturen und Kombinationen davon aufweist.

13. Implantat (100) nach einem der vorhergehenden Ansprüche, **dadurch gekennzeichnet, dass** das Implantat (100), insbesondere der überhöhte Formkörper (105) und/oder die zumindest eine zusätzliche Überhöhung (140a;b) aus einem natürlichen Polymer bzw. Biopolymer und/oder synthetischen Polymer, vorzugsweise ausgewählt aus der Gruppe bestehend aus Proteine, Polysaccharide, Polyhydroxyalkanoate, Polyvinylalkohol, Polyurethane und Kombinationen davon, gebildet ist.

14. Implantat (100) nach einem der vorhergehenden Ansprüche, **dadurch gekennzeichnet, dass** das Implantat (100), insbesondere der überhöhte Formkörper (105) und/oder die zumindest eine zusätzliche Überhöhung (140a;b), mit Körperzellen, insbesondere autologen Körperzellen, vorzugsweise aus der Gruppe bestehend aus Chondrozyten, Chondroblasten, Synovialzellen, Fibroblasten, Vorläuferzellen davon, Stammzellen davon und Kombinationen davon, inokuliert ist.

15. Implantat (100) nach einem der vorhergehenden Ansprüche, **dadurch gekennzeichnet, dass** das Implantat (100), insbesondere der überhöhte Formkörper (105) und/oder die zumindest eine zusätzliche Überhöhung (140a;b), einen Anteil eines faserförmigen Materials aufweist, wobei das Implantat (100) vorzugsweise einen Anteil an dem faserförmigen Material zwischen 2 und 80 Gew.-%, insbesondere 10 und 30 Gew.-%, vorzugsweise 15 und 25 Gew.-%, aufweist, bezogen auf das Gesamtgewicht des Implantats (100).

16. Chirurgisches Set zur Rekonstruktion von Meniskusdefekten oder Meniskusteildefekten, umfassend zumindest ein Implantat (100) nach einem der vorhergehenden Ansprüche.

## Claims

1. Implant (100) for the reconstruction of meniscus defects or partial meniscus defects, comprising an elevated molding (105) with a femoral condylar contact area (110), a tibial condylar contact area (120) and an articular capsule contact area (130), in particular in the manner of an artificial meniscus or artificial partial meniscus with a contour of the outer area that is essentially similar to a natural meniscus or partial meniscus, wherein at least one additional elevation (140a; b) is formed on the outer area of the elevated molding (105), **characterized in that** the elevated molding (105) is of an open-pore form in the region of the articular capsule contact area (130) and is of a closed-pore form in the region of the femoral condylar contact area (110) and/or tibial condylar contact area (120).

2. Implant (100) according to claim 1, **characterized in that** the implant (100) has a compound structure comprising the elevated molding (105) and the at least one additional elevation (140a; b), in particular in the manner of a composite.

3. Implant (100) according to claim 1 or 2, **characterized in that** the at least one additional elevation (140a; b) is formed on the femoral condylar contact area (110), tibial condylar contact area (120) and/or articular capsule contact area (130), preferably on the femoral condylar contact area (110) and/or tibial condylar contact area (120), of the elevated molding (105).

4. Implant (100) according to any of the preceding claims, **characterized in that** the at least one additional elevation (140a; b) is formed from a different material than the elevated molding (105).

5. Implant (100) according to any of the preceding claims, **characterized in that** the at least one additional elevation (140a; b) is formed from a resorbable material, in particular a more quickly resorbable material than the elevated molding (105).

6. Implant (100) according to any of the preceding claims, **characterized in that** layers of the at least one additional elevation (140a; b) form a resorption gradient, the resorption rate of the layers preferably increasing essentially continuously from a side of the at least one additional elevation (140a; b) that is facing the elevated molding (105) to an opposite side of the at least one additional elevation (140a; b).

7. Implant (100) according to any of the preceding claims, **characterized in that** the at least one additional elevation (140a; b) is formed from a more flexible, in particular softer, with preference more elastic, material than the elevated molding (105).

8. Implant (100) according to any of the preceding claims, **characterized in that** the at least one additional elevation (140a; b) is of a porous form, in particular of an open-pore form, wherein the elevated molding (105) is of an open-pore form in the region of its articular capsule contact area (130).

9. Implant (100) according to any of the preceding claims, **characterized in that** the at least one additional elevation (140a; b) contains closed pores.

10. Implant (100) according to any of the preceding claims, **characterized in that** open pores of the implant (100), in particular of the elevated molding (105) and/or the at least one additional elevation (140a; b), are at least partially filled with a resorbable and/or viscous material.

11. Implant (100) according to any of the preceding claims, **characterized in that** the implant (100), in particular the elevated molding (105) and/or the at least one additional elevation (140a; b), is of a disk-shaped, semicircular, sickle-shaped or crescent-shaped form.

12. Implant (100) according to any of the preceding claims, **characterized in that** the implant (100), in particular the elevated molding (105) and/or the at least one additional elevation (140a; b), has anchorage elements (150), preferably selected from the group comprising anchor hooks, anchor flukes, anchor pins, anchor screws, filamentary structures and combinations thereof.

13. Implant (100) according to any of the preceding claims, **characterized in that** the implant (100), in particular the elevated molding (105) and/or the at least one additional elevation (140a; b), is formed from a natural polymer or biopolymer and/or synthetic polymer, preferably selected from the group comprising proteins, polysaccharides, polyhydroxyalkanoates, polyvinyl alcohol, polyurethanes and combinations thereof.

14. Implant (100) according to any of the preceding claims, **characterized in that** the implant (100), in particular the elevated molding (105) and/or the at least one additional elevation (140a; b), is inoculated with body cells, in particular autologous body cells, preferably from the group comprising chondrocytes, chondroblasts, synovial cells, fibroblasts, precursor cells thereof, stem cells thereof and combinations thereof.

15. Implant (100) according to any of the preceding claims, **characterized in that** the implant (100), in particular the elevated molding (105) and/or the at least one additional elevation (140a; b), has a proportion of a fibrous material, wherein the implant (100) preferably has a proportion of the fibrous material of between 2 and 80% by weight, in particular 10 and 30% by weight, preferably 15 and 25% by weight, with respect to the overall weight of the implant (100).

16. Surgical set for the reconstruction of meniscus defects or partial meniscus defects, comprising at least one implant (100) according to any of the preceding claims.

## Revendications

1. Implant (100) destiné à la reconstruction des défauts du ménisque ou des défauts d'une partie du ménisque, comprenant un corps moulé (105) surélevé avec une surface de contact fémorale avec les condyles (110), une surface de contact tibiale avec les condyles (120) et une surface de contact avec la capsule articulaire (130), lequel implant (100) se présente en particulier sous la forme d'un ménisque artificiel ou d'une partie de ménisque artificiel avec un contour de la surface extérieure qui est, pour l'essentiel, le même que celui d'un ménisque naturel, respectivement d'une partie de ménisque naturel, selon lequel tout au moins une surélévation (140a ; b) supplémentaire est conçue sur la surface extérieure du corps moulé (105) surélevé, **caractérisé en ce que** le corps moulé (105) surélevé est conçu avec des pores ouverts dans la zone de la surface de contact avec la capsule articulaire (130) et est conçu avec des pores fermés dans la zone de la surface de contact fémorale avec les condyles (110) et/ou de la surface de contact tibiale avec les condyles (120).

2. Implant (100) selon la revendication 1, **caractérisé en ce que** l'implant (100) possède une structure composite à partir du corps moulé (105) surélevé et de la tout au moins une surélévation (140a ; b) supplémentaire, qui se présente, en particulier, sous la forme d'un composite.

3. Implant (100) selon la revendication 1 ou 2, **caractérisé en ce que** la tout au moins une surélévation (140a ; b) supplémentaire est conçue sur la surface de contact fémorale avec les condyles (110), sur la surface de contact tibiale avec les condyles (120) et/ou sur la surface de contact avec la capsule articulaire (130), de préférence sur la surface de contact fémorale avec les condyles (110) et/ou sur la surface de contact tibiale avec les condyles (120), du corps moulé (105) surélevé.

4. Implant (100) selon l'une des revendications précédentes, **caractérisé en ce que** la tout au moins une surélévation (140a ; b) supplémentaire est formée à partir d'un autre matériau que le corps moulé (105) surélevé.

5. Implant (100) selon l'une des revendications précédentes, **caractérisé en ce que** la tout au moins une surélévation (140a ; b) supplémentaire est formée à partir d'un matériau pouvant être résorbé, en particulier à partir d'un matériau pouvant être résorbé plus rapidement que le corps moulé (105) surélevé.

6. Implant (100) selon l'une des revendications précédentes, **caractérisé en ce que** des couches de la tout au moins une surélévation (140a ; b) supplémentaire forment un gradient de résorption, selon lequel, de préférence, la vitesse de résorption des couches augmente pour l'essentiel en continu depuis une face de la tout au moins une surélévation (140a ; b) supplémentaire, laquelle face est tournée vers le corps moulé (105) surélevé, vers une face de la tout au moins une surélévation (140a ; b) supplémentaire, laquelle face se trouve à l'opposé.

7. Implant (100) selon l'une des revendications précédentes, **caractérisé en ce que** la tout au moins une surélévation (140a ; b) supplémentaire est formée à partir d'un matériau plus flexible, en particulier plus souple, de préférence plus élastique que le corps moulé (105) surélevé.

8. Implant (100) selon l'une des revendications précédentes, **caractérisé en ce que** la tout au moins une surélévation (140a ; b) supplémentaire est conçue de manière poreuse, en particulier avec des pores ouverts, selon lequel le corps moulé (105) surélevé est conçu avec des pores ouverts dans la zone de sa surface de contact avec la capsule articulaire (130).

9. Implant (100) selon l'une des revendications précédentes, **caractérisé en ce que** la tout au moins une surélévation (140a ; b) supplémentaire contient des pores fermés.

10. Implant (100) selon l'une des revendications précédentes, **caractérisé en ce que** des pores ouverts de l'implant (100), en particulier du corps moulé (105) surélevé et/ou de la tout au moins une surélévation (140a ; b) supplémentaire, sont remplis, tout au moins en partie, avec un matériau pouvant être résorbé et/ou visqueux.

11. Implant (100) selon l'une des revendications précédentes, **caractérisé en ce que** l'implant (100), en particulier le corps moulé (105) surélevé et/ou la tout au moins une surélévation (140a ; b) supplémentaire, est conçu en forme de disque, en forme de demi-cercle, en forme de croissant ou en forme de demi-lune.

12. Implant (100) selon l'une des revendications précédentes, **caractérisé en ce que** l'implant (100), en particulier le corps moulé (105) surélevé et/ou la tout au moins une surélévation (140a ; b) supplémentaire, présente des éléments d'ancrage (150), de préférence sélectionnés parmi le groupe constitué par les crochets d'ancrage, les flèches d'ancrage, les tiges d'ancrage, les vis d'ancrage, les structures filiformes et des combinaisons de ces éléments.

13. Implant (100) selon l'une des revendications précédentes, **caractérisé en ce que** l'implant (100), en particulier le corps moulé (105) surélevé et/ou la tout au moins une surélévation (140a ; b) supplémentaire, est formé à partir d'un polymère naturel, respectivement à partir d'un biopolymère naturel et/ou d'un polymère de synthèse, de préférence sélectionné parmi le groupe constitué par les protéines, les polysaccharides, les polyhydroxyalcanoates, l'alcool polyvinylique, les polyuréthanes et des combinaisons de ces éléments.

14. Implant (100) selon l'une des revendications précédentes, **caractérisé en ce que** l'implant (100), en particulier le corps moulé (105) surélevé et/ou la tout au moins une surélévation (140a ; b) supplémentaire, est inoculé avec des cellules du corps, en particulier des cellules autologues du corps, de préférence sélectionnées parmi le groupe constitué par les chondrocytes, les chondroblastes, les cellules synoviales, les fibroblastes, les cellules progénitrices de ces cellules, les cellules souches de ces cellules et des combinaisons de ces cellules.

15. Implant (100) selon l'une des revendications précédentes, **caractérisé en ce que** l'implant (100), en particulier le corps moulé (105) surélevé et/ou la tout au moins une surélévation (140a ; b) supplémentaire, présente une proportion d'un matériau fibreux, selon lequel l'implant (100) présente de préférence une proportion de ce matériau fibreux comprise entre 2 % et 80 % en poids, en particulier comprise entre 10 % et 30 % en poids, de préférence comprise entre 15 % et 25 % en poids, mesurée par rapport au poids total de l'implant (100).

16. Ensemble de chirurgie destiné à la reconstruction des défauts du ménisque ou des défauts d'une partie du ménisque, comprenant tout au moins un implant (100) selon l'une des revendications précédentes.
